# EUROPEAN PATENT SPECIFICATION

(11) **EP 1 416 278 B1**
(45) Date of publication and mention of the grant of the patent: **24.11.2004**
(21) Application number: 02024030.5
(22) Date of filing: 28.10.2002
(51) Int. Cl.: G01N 33/574

(54) **Method for improved diagnosis of dysplasias**
Verfahren zur verbesserten Diagnose von Dysplasien
Procédé pour un diagnostic amélioré de la dysplasie

(43) Date of publication of application: 06.05.2004
(73) Proprietor: MTM Laboratories AG, 69120 Heidelberg (DE); Dako-Cytomation Denmark A/S, 2600 Glostrup (DK)
(72) Inventor: Trunk-Gehmacher, Marcus, 69115 Heidelberg (DE); Reichert, Anja, 69226 Nussloch (DE); Batrla,Richard, 69120 Heidelberg (DE); Ridder, Rüdiger, 69198 Schriesheim (DE)

(56) References cited:
- RIETHDORF LUTZ ET AL: "Human papillomaviruses, expression of p16INK4A, and early endocervical grandular neoplasia." HUMAN PATHOLOGY, vol. 33, no. 9, September 2002 (2002-09), pages 899-904, XP009008281 ISSN: 0046-8177
- BEPLER G ET AL: "Mcm2: A novel diagnostic marker for early detection of premalignant lesions of the lung." PROCEEDINGS OF THE AMERICAN ASSOCIATION FOR CANCER RESEARCH ANNUAL, vol. 42, March 2001 (2001-03), page 356 XP001146358 92nd Annual Meeting of the American Association for Cancer Research;New Orleans, LA, USA; March 24-28, 2001, March, 2001 ISSN: 0197-016X
- VON KNEBEL DOEBERITZ MAGNUS: "New markers to identify HPV-transformed dysplastic cervical epithelia." ACTA CYTOLOGICA, vol. 46, no. 1 Supplement, January 2002 (2002-01), page 156 XP009008277 14th International Congress of Cytology;Amsterdam, Netherlands; May 27-31, 2001, January-February, 2002 ISSN: 0001-5547
- SIMONART THIERRY ET AL: "Antiproliferative and apoptotic effects of iron chelators on human cervical carcinoma cells." GYNECOLOGIC ONCOLOGY, vol. 85, no. 1, April 2002 (2002-04), pages 95-102, XP002236573 April, 2002 ISSN: 0090-8258
- KLAES R ET AL: "Overexpression of p16(INK4A) as a specific marker for dysplastic and neoplastic epithelial cells of the cervix uteri." INTERNATIONAL JOURNAL OF CANCER. JOURNAL INTERNATIONAL DU CANCER. UNITED STATES 15 APR 2001, vol. 92, no. 2, 15 April 2001 (2001-04-15), pages 276-284, XP002236574 ISSN: 0020-7136

## Description

The present invention relates to a method for improved diagnosis of dysplasias based on simultaneous detection of INK4a gene products and at least one marker for cell proliferation. Particularly the present invention provides a method for discriminating dysplastic cells over-expressing INK4a gene products from cells over-expressing INK4a gene products without being dysplastic by detection of a marker suitable for characterising the proliferation properties of the respective cell. The characterisation of the proliferation properties may comprise the detection of a marker or a set of markers characteristic for active cell proliferation and/or a marker or a set of markers characteristic for retarded or ceased cell proliferation. The method presented herein thus enables for a specific diagnosis of dysplasias in histological and cytological specimens.

The detection of the over-expression of p16^{INK4a} in biological samples has proven as a useful marker in the detection of anogenital lesions, such as carcinoma of the uterine cervix (see WO00/01845; Klaes et al., Int. J. Cancer: 92, 276-284 (2001)). The method based on p16^{INK4a}-specific immuno-chemical staining allows for a sensitive and specific identification of dysplastic cells in tissue section and in cytological samples.

In immuno-histochemical examinations of tissues dysplastic and neoplastic cells can be stained using a p16^{INK4a} specific antibody mediated staining procedure. The histological diagnosis of neoplastic lesions can thus be supported by a staining procedure based on a molecular marker characteristic for transformation of cells in anogenital lesions. The diagnosis, whether or not cells are neoplastic, in these procedures is not solely based on the p16^{INK4a} specific staining, but does also rely on the histological information.

This is due to the fact, that in about 20 - 30 % of samples metaplastic cells show some immuno-reactivity with p16^{INK4a} specific antibodies, and thus are stained in the course of the procedures. Yet the staining pattern yielded from these metaplastic cells differs from the pattern, which renders from neoplastic lesions. Metaplastic cells give rise to a patchy or focal staining pattern, whereas neoplastic lesions give rise to diffuse staining pattern. Moreover the staining intensities of metaplastic cells are predominantly less than that of neoplastic cells.

The common methods used in screening tests for the early detection of dysplasias and/or neoplasias do not employ histology based tests, but do rather rely on cytological testing procedures. Yet especially in cases, when there is no histological information available concerning the architecture of tissues, such as for example in cytological examinations, testing for p16^{INK4a} over-expression alone may lead to false positive results. This is due to the fact, that those fractions of metaplastic cells expressing p16^{INK4a} at detectably elevated levels, may not be differentiated by means of a histologic criteria.

The percentage of cells showing over-expression of p16^{INK4a} increases in the course of emergence of dysplasias. So in neoplastic or pre-neoplastic stages, when only a restricted population of neoplastic or pre-neoplastic cells is present in samples the immuno-reactivity of p16^{INK4a} may be weak. This weak immuno-reactivity may be of about the level as the level caused by metaplastic cells. In later stages of dysplasias the overall immuno-reactivity of p16^{INK4a} is stronger and so neoplastic lesions are easily discernible from metaplasias even in a cytological testing format. This might lead to cases, where the presence of metaplastic cells expressing p16^{INK4a} might be confused with the presence of neoplastic cells, and thus produces a false positive result.

Especially in screening tests, where the detection of early stages of neoplasias is desirable this condition is quite unpleasant. This is especially true, as the p16^{INK4a} based diagnosis has proven to be a valuable tool in histological examinations and the application in cytological based screening procedures would be able to enhance these established procedures.

To reduce false positive results in cytological testing formats and so to further enhance the fidelity of the p16^{INK4a} mediated diagnosis of anogenital lesions a method for discriminating the metaplasias from neoplastic and dysplastic lesions would be desirable. A method for the discrimination of metaplasias from neoplastic and pre-neoplastic lesions is provided within the embodiments claimed according to the present invention.

For supporting the discrimination of metaplasias from neoplastic lesions in testing procedures based on the over-expression of p16^{INK4a} a marker molecule would be desirable, that is expressed in neoplastic and/or pre-neoplastic cells and tissues and, which is not expressed simultaneously with INK4a gene products in one single metaplastic cells.

A solution to the problem present in the art is provided by the methods claimed according to this invention. In the course of the experiments leading to the present invention the inventors have found, that a combination of detection of the presence or absence and/or the level of p16^{INK4a} in combination with at least one marker for cell proliferation such as e.g. Ki67, Ki-S2, mcm5 or mcm2 may solve the problem present in the art.

In the art a couple of documents pertaining to the use of a combination of molecular markers for improved diagnosis of dysplasias is presented. In WO0208764 a method for improved diagnosis of cervical malignancies is disclosed using a combination of an HPV marker and a marker for cell proliferation or viral activity. p16^{INK4a} is mentioned in the context of this invention as a viral activity marker to be combined with HPV markers.

In EP1217377 a method for automated detection of cervical malignancies is disclosed mediated by detection of more than one marker molecules. Some defined marker combinations are named within the document. There is no disclosure in this document relating to the choice of suitable markers for a combination. The purpose of the combination in this application is improved fidelity of the automated analysis of staining patterns in biological cytological specimens. This document mentions combination of p16^{INK4a} with other tumor markers.

WO02059616 discloses a method for detection of cell-cycle disturbances for improved diagnosis of cervical malignancies. The document discloses, that dysplastic cells exhibit disturbances in cell cycle control and may thus be identified by means of detection of cyclin E type proteins together with post G1 substances in cells.

Jeffrey Keating in "Ki67, Cyclin E, and p16^{INK4a} Are Complimentary Surrogate Biomarkers for human papilloma Virus-Related Cervical Neoplasia" (American Journal of Surgical Pathology 25(7): 884-891, 2001) discloses the complementarity of the use of p16^{INK4a}, Ki67 and Cyclin E in the course of diagnosis of cervical dysplasias. The document refers to the problems of each single marker in the detection of dysplasias and states, that p16^{INK4a} in combination with Cyclin E may be suitable to overcome the drawbacks of the single marker molecules especially when cytological specimens are under examination. No disclosure teaching use of p16^{INK4a} in concert with a marker characteristic for proliferating cells such as Ki67 is given. The document does not teach the combination of p16^{INK4a} for use in diagnostic methods; moreover the disclosure pertains to a restricted use of Ki67 in cervical differential diagnosis and thus teaches away from the use of this marker in diagnosis of cervical malignancies.

In the art there is no disclosure teaching the use of a combination of p16^{INK4a} with a marker characteristic for cell proliferation for use in a diagnostic method for improved discrimination of p16^{INK4a} positive non-dysplastic cells from p16^{INK4a} positive dysplasias. WO02059616 does not give a hint as to the use of p16^{INK4a} in the detection of dysplastic cell proliferation. EP1217377 does not teach a purpose for the combination of tumor markers in the course of the detection than the automation of the analysis process. There is no disclosure pertaining to the advantage of combinations for specific discrimination purposes such as discrimination of dysplastic cells from e.g. metaplastic cells in cervical specimens.

The inventors of the present invention sought to overcome the drawback present in the art that p16^{INK4a}, over-expressed in various dysplasias, may also be detected in some other non-dysplastic cells. The discrimination between non-dysplastic p16^{INK4a} positive cells and dysplastic cells over-expressing p16^{INK4a} may be based on the proliferation characteristics of the respective cells. In normally controlled cells p16^{INK4a} inhibits cdk4 and thus inhibits proliferation. In contrast in dysplastic cells this regulation is impaired. Thus p16^{INK4a} does despite it's uncommonly high expression level not lead to an inhibition of the cell proliferation.

The inventors of the present invention found that dysplastic cells may be discriminated from cells exhibiting controlled cell proliferation by the simultaneous detection of p16^{INK4a} with a marker characteristic for cell proliferation. Due to the fact, that in normal cells elevated levels of p16^{INK4a} inhibit cell proliferation, cells over-expressing p16^{INK4a} may be classified as being dysplastic provided they are exhibiting the characteristics of active cell proliferation.

The present invention relates to a method for discrimination of neoplastic, pre-neoplastic and/or dysplastic lesions from non-dysplastic cells showing elevated levels of p16^{INK4a}, in biological samples in a histological or cytological testing procedure based on the detection of the presence or absence of cells expressing p16^{INK4a} gene simultaneously with cell proliferation markers in said biological samples. Suitable markers for cell proliferation may be e.g. Ki67, Ki-S2, Ki-S5, mcm5 or mcm2. In one embodiment of the invention protein or mRNA of the markers for cell proliferation may serve as a marker for discrimination of metaplasias from early dysplastic or pre-neoplastic lesions in samples.

It is one aspect of the present invention to provide a method for discriminating dysplastic cells over-expressing INK4a gene products from other cells expressing INK4a gene products at a detectable level in biological samples comprising determining in a cytological or histological testing procedure the co-expression of at least two marker molecules in at least one single cell, wherein at least one marker molecule is an expression product encoded by the INK4a gene and at least one further marker molecule is a cell proliferation marker, wherein the over-expression of at least one INK4a gene product and expression of at least one marker for active cell proliferation at an immunochemically detectable level within said single cell is indicative of the dysplastic state of the cell and wherein the over-expression of at least one INK4a gene product and expression of at least one marker for senescence, terminal differentiation of cells, apoptosis or cell cycle arrest at a detectable level within said single cell is indicative of the non-dysplastic state of the cell.

A second aspect of the present invention relates to a test kit for determination of dysplasias in samples according to the method disclosed herein and comprising the components as defined in the kit claims.

During the experiments leading to the present invention it was found, that under certain circumstances non-dysplastic cells may show immuno-reactivity for p16^{INK4a}. The inventors found that these cells exhibiting ordered control of cell proliferation in response to the elevated levels of the INK4a gene products are subject to growth arrest. Thus these individual cells do not show immuno-reactivity for markers of cell proliferation. In contrast transformed cells over-expressing p16^{INK4a} exhibit dysregulated control of cell proliferation and do not respond to the elevated level of p16^{INK4a} by cessation of proliferation. Thus these dysplastic cell show simultaneous expression of p16^{INK4a} with markers for cell proliferation. The inventors thus found out, that a simultaneous detection of p16^{INK4a} with markers for cell proliferation may serve to discriminate dysplastic cells from arrested cells over-expressing p16^{INK4a} such as e.g. metaplastic cells.

The discovery that p16^{INK4a} over-expressed in various dysplasias may also be detected in some other non-dysplastic cells led the inventors of the presented method to establish a technique for discrimination between non-dysplastic p16^{INK4a} positive cells and dysplastic cells over-expressing p16^{INK4a} based on the proliferation characteristics of the respective cells. Whereas in normally controlled cells p16^{INK4a} inhibits cdk4 and thus inhibits cell proliferation, this is not true for dysplastic cells. Thus in dysplastic cells p16^{INK4a} does despite it's uncommonly high expression level not lead to an inhibition of the cell proliferation.

The method disclosed herein is based on the fact that dysplastic cells may be discriminated from cells exhibiting normally controlled cell proliferation by the simultaneous detection of a INK4a gene product such as e.g. p16^{INK4a} with a marker characteristic for cell proliferation.

The term marker as well as marker molecules in general shall be used herein to pertain to proliferation marker gene expression products as well as to INK4a gene expression products.

The denominations given throughout this text for genes may in part relate to the genes or proteins as they have been discovered from any organism. In the context of the present invention this denomination shall confer to the respective homologue of the named markers in the organism which is particularly in question for a method as disclosed herein. In certain embodiments of the present invention this organism is a mammal and in one embodiment may be a human being. Such in one embodiment of the present invention the named markers shall be the human homologues of the respective denominated ones.

Generally throughout the text the term "(cell) proliferation marker" or "marker for cell proliferation" in the various grammatical forms is used to denominate proteins as well as nucleic acid markers. In case the protein name of a marker such as e.g. "replication protein" is used herein, this use shall be understood to be metonymically and pertain as well to the protein as to the nucleic acid marker molecules encoding the particular protein

A marker useful according to the present invention may be any molecule transcribed from a gene or any molecule translated from such a transcript. Thus gene product as used in the context of the present invention may comprise polynucleotides such as e.g. DNA or RNA and polypeptides such as proteins, proteoglycans, peptides etc. Expression product(s) as used in the context of the present invention shall comprise any transcript of a gene locus in forward or reverse direction including any reading frames, splicing variants. Expression products as used herein shall thus comprise any alternative products encoded by the nucleic acids of a particular gene locus.

INK4a encoded gene-products as used in the context of the present invention shall be any mRNA transcribed from the INK4a gene locus or any polypeptide translated from such an mRNA. In one embodiment of the invention the expression products encoded by the INK4a gene may exhibit molecular weights of about 5 to 40 kDa or any value in between and preferably of about 10 to 20 kDa or any value in between and most preferably of about 14 to about 19 kDa or any value in between respectively.

INK4a gene-products suitable for the method according to the present invention may comprise e.g. gene-products such as e.g. p16^{INK4a} and p14ARF.

The term "(cell) proliferation marker" or "marker for cell proliferation" as used in the context of the present invention shall comprise any marker molecule known in the art to be characteristic for the proliferation status of cells. The proliferation status may e.g. be a status of actively proliferating cells, of retarded cell proliferation, of arrested cell proliferation, of senescent cells, of terminally differentiated cells, of apoptosis etc. In one embodiment of the invention the cell proliferation marker is a marker molecule characteristic for active cell proliferation. In another embodiment of the invention the proliferation marker molecule may be a molecule characteristic for arrested, terminally differentiated, senescent or apoptotic cells.

In certain embodiments proliferation markers for use in the context of the present invention may comprise genes engaged in the DNA replication such as e.g. proteins of the pre-initiation complex or of the replication fork. Such molecules may e.g. comprise helicases, such as eucaryotic helicase or MCM proteins (MCM2, MCM3, MCM4, MCM5, MCM6, MCM7), protein TP as disclosed in WO0050451 and WO0217947 (also denominated HELAD1, Pomfil2, Unc-53), kinases or phosphatases engaged in the replication process such as e.g. CDC6. CDC7 protein kinase, Dbf4, CDC14 protein phosphatase, CDC45 and MCM10. Furthermore proliferation markers may comprise proteins engaged in the processive replication fork such as e.g. PCNA or DNA polymerase delta, replication protein A (RPA), replication factor C (RFC), FEN1.

In other embodiments the proliferation markers may comprise molecules necessary for the maintenance of cell proliferation such as Ki67. Ki-S5 or Ki-S2. In this embodiment proteins may be e.g. present throughout the whole cell cycle. They are useful for performing a method according to the present invention provided they are characteristic for active cell proliferation and are not significantly expressed in arrested, terminally differentiated, apoptotic or senescent states of cells. Ki67, Ki-S2 and ki-S5 as used herein shall denominate the protein marker molecules detected by the respective antibodies as well as the nucleic acids encoding these antigens.

In another embodiment the cell proliferation markers for use in a method according to the present invention may be a marker molecule characteristic for retarded or ceased cell proliferation such as e.g. a senescence marker, a cell cycle arrest marker, a marker characteristic for terminally differentiated cells or an apoptosis marker. Such molecules comprise e.g. p21, p27, Caspases, BAD, CD95, fas-ligand, parp-proteins etc.

Discrimination as used in the context of the present invention shall comprise an assessment whether a sample is to be classified in one or another way. In one embodiment of the invention the discrimination pertains to the assessment of a tissue or components thereof being dysplastic or being not dysplastic. Thus the discrimination as used herein is a judgement about the growth properties of cells in a biological sample.

In one embodiment of the present invention the discrimination comprises the detection of expression of an INK4a gene product simultaneously with the detection of expression of a marker characteristic for active cell proliferation. In this case cells co-expressing both marker molecules are to be classified as being dysplastic.

In another embodiment of the present invention the discrimination comprises the detection of an INK4a gene product simultaneously with the detection of expression of a marker characteristic for arrested, ceased or retarded cell proliferation. In this case cells co-expressing both marker molecules are to be classified as being non-dysplastic.

In certain embodiments of the present invention it will be useful to detect the presence or absence and or the level of more than two marker molecules. In one embodiment one INK4a gene expression product will be detected in concert with two or more markers for cell proliferation. This may be useful to enhance the identification of proliferation properties of the INK4a gene product expressing cells in samples. Some proliferation markers are restricted to specific phases of the cell cycle or are present in low abundance in cells. Due to this fact in some cases the detection of proliferating cells expressing INK4a gene products may be improved by detection of two or more proliferation markers.

In such cases e.g. one proliferation being expressed during the whole proliferative cell cycle may be detected simultaneously to markers being characteristic for specific cells cycle phases. E.g. Ki67, Ki-S5 or Ki-S2 may be detected together with mcm5, mcm2, PCNA, rpA, rfC etc.. In other cases e.g. proteins engaged in the DNA replication may be detected together with Ki67, Ki-S5 or Ki-S2. In yet another case Ki67 may be detected together with Ki-S2. It must be understood, that these examples are intended to exemplify combinatorial possibilities and shall not be comprehensive, so that various other combinations of proliferation markers are likewise useful and suitable in the procedure of the method according to the present invention.

As the case may be, combination of two or more cell proliferation marker molecules may be applied for a method as disclosed herein. In another embodiment two or more marker molecules being detectable over larger stretches of the cell cycle or even over the whole cell cycle or actively proliferating cell may be detected in concert in a method as disclosed herein. A combination of more than one cell proliferation marker molecules may generally be useful for improving the sensitivity of the detection of the proliferation characteristics of cells.

In certain embodiments of the present invention a combination may furthermore comprise other marker molecules such as senescence marker molecules, markers for arrested cells, markers for terminally differentiated cells, markers for apoptotic cells, markers for viral infection or for viral activity in cells or cell cycle regulatory proteins as markers. In certain embodiments in connection with dysplasias being associated with HPV infection a detection of HPV associated marker molecules or markers for viral activity may be of use for a detection of a dysplasia. The methods useful for detection of detection of HPV infection in samples are known to those of skill in the art. These methods may comprise methods employing probes specific for HPV agents or may employ nucleic acid amplification reactions. The detection of a viral infection may be carried out simultaneously or subsequently to the detection of the INK4a and proliferation marker molecules.

Dysplastic as used in the context of the present invention shall refer to dysplasias from mild to severe dysplasias and their precursory stages, as well as carcinoma such as carcinomas in situ or invasive carcinomas and disseminated tumor cells. Thus dysplastic as used herein shall also comprise early and precursory stages of dysplasias and carcinomas.

Cells over-expressing INK4a gene products without being dysplastic (non-dysplastic as used herein) as mentioned herein may e.g. comprise metaplastic cells, senescent cells, terminally differentiated cells or cells, that in certain stages of the cell cycle exhibit elevated levels of the INK4a gene products. In certain cells elevated levels of INK4a gene products may even be effected as a response to external signals such as hormones, transmitters etc. In one embodiment of the present invention the non-dysplastic cells over-expressing INK4a gene products comprise e.g. metaplastic cells, endometrial cells etc.

The method for detection of the expression level of the INK4a encoded gene-products and/or the proliferation marker gene products according to the present invention is any method, which may (but need not) be e.g. suited to detect even very small amounts of specific biological molecules in biological samples. The detection reaction according to the present invention is a detection either on the level of nucleic acids or on the level of polypeptides.

A marker molecule is said to be detectable as used in the context of the present invention, provided the marker may be detected in the course of suitable detection procedure such as e.g. in-situ-hybridization, immuno-chemical staining, hybrid capture assay etc.. The level of expression of a marker molecule may be made detectable using suitable reporter reactions such as e.g. a chromogenic or fluorescence based immuno-chemical staining or in-situ-hybridization procedure for microscopic or automated analysis. Suitable methods for enhancing the reporter signal known to those of skill in the art may be applied in the course of a method according to the present invention. Thus the marker is said to be detectable in a case where the staining supersedes the respective background staining inherently obtained in the immuno-chemical staining procedure so as to produce significant staining results.

The marker molecules may be detected using reagents that specifically recognise these molecules. The detection reaction for the INK4a gene-products and/or the proliferation marker gene products may comprise one or more reactions with detecting agents either recognising the initial marker molecules or recognising the prior molecules used to recognise other molecules.

In certain embodiments of the present invention two or more probes may be used for the detection of one single marker molecule. For example two or more different binding agents (e.g. antibodies) or oligonucleotide probes directed against one single marker molecule (as the case may be against different epitopes or different sequences) may be used in the course of the method as disclosed herein.

The detection of the different gene products may be performed in one reaction vessel or containment or in different containments simultaneously or subsequently in time. Thus the different gene products may be detected simultaneously in one cell co-expressing both products. Otherwise cells co-expressing the gene products may be used for separated detection reaction (separated in space or in time) to detect each a single marker in the cells. In another embodiment there might be cells expressing one or the other marker. The detection of the marker molecules in the different cells may as well be performed simultaneously or separately in time and/or space.

The detection reaction further may comprise a reporter reaction indicating the presence or absence and/or the level of the marker molecule gene-products. The reporter reaction may be for example a reaction producing a coloured compound, a bioluminescence reaction, a fluorescence reaction, generally a radiation emitting reaction etc..

In certain embodiments, different marker molecules may be recognised by agents, that produce different reporter signals, so that the signals referring to marker molecules could be distinguished. In one preferred embodiment of the invention the detection of the expression of the two or more INK4a gene-products and/or proliferation marker gene products is carried out simultaneously. In this case the reporter reaction may for example employ different fluorescent labels for the different molecules detected.

However within the context of the present invention it must not necessarily be answered whether the one or the other proliferation marker or INK4a marker gene product is expressed in the cells. In certain embodiments the question is whether any proliferation marker and/or INK4a gene product is expressed. Such in the course of the experiments a procedure may be chosen, that gives the same fluorescence signal as indication of the presence of a proliferation marker. This procedure is suitable to improve sensitivity of the detection of the cell proliferation characteristics (different markers characteristic for active cell proliferation). As the case may be the procedure may be applied as to render one detectable signal for three, four or even more marker molecules being characteristic for cell proliferation. Analogous the same may under certain circumstances be true for the INK4a gene expression products. It must be understood, that as the case may be different staining signals for different proliferation marker molecules may be desirable. The procedures may be applied to the necessities of the respective experiment.

In certain embodiments of the present invention a combination of one or more (e.g. two different) INK4a gene products may be detected with a combination of one or more e.g. a set of two, a set of three, a set of four, a set of five or a set of even more markers for cells proliferation. As the case may be the detection of the marker molecules for cell proliferation may render only one reporter signal. In other cases each single marker for cell proliferation may render a specific reporter signal or groups of marker molecules may render specific reporter signals.

Signals for the indication of the presence of immuno-reactivity may be chromogenic signals produced by various methods known in the art. Alternatively or even in combination fluorescent signals may be used. Suitable reporter signals comprise fluorescent labels such as fluorescein, rhodamin etc.

Applicable formats for the detection reaction according to the present invention may be, blotting techniques, such as Western-Blot, Southern-blot, Northern-blot, immuno-cytochemical or immuno-histochemical procedures. The blotting techniques are known to those of ordinary skill in the art and may be performed for example as electro-blots, semidry-blots, vacuum-blots or dot-blots. Immuno-cyto/histochemical staining procedures are known to those of skill in the art and may comprise binding agent mediated detection of polypeptides as well as in situ hybridisation techniques. Both different techniques may even be applied simultaneously. In certain embodiment hybrid capture of nucleic acids may be used for the detection. Amplification reaction may also be applicable for the detection of e.g. nucleic acid molecules.

In one embodiment of the invention the detection of the level of INK4a and/or proliferation marker gene-products is carried out by detection of the respective nucleic acids (e.g. mRNA) or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognising and binding to said nucleic acids. In one embodiment in situ hybridisation of oligonucleotide probes to nucleic acids in a sample may be used for the detection of expression products or markers.

Probes as used in the context of the present invention may be any agent binding specifically to a molecule. In the case of nucleic acids a probe may be an oligonucleotide hybridising to a particular sequence. In one embodiment the probe may be e.g. a primer. In the case of the detection of polypeptides or proteins the probe as used herein may be e.g. a binding agent such as an antibody. In certain embodiments of the present invention the probes may be detectably labelled. The label may be selected from the group comprising a radioisotope, a bioluminescent compound, a chemiluminescent compound, a fluorescent compound, a metal chelate, or an enzyme. Probes may be applied in any detection procedure known in the art e.g. in the course of an in situ hybridisation procedure, in the course of hybrid capture assays, in the course of immuno-chemical staining reaction, in the course of blotting techniques etc..

This method may be performed as well in vitro as directly in situ for example in the course of a detecting staining reaction. Another way of detecting the marker mRNAs in a sample performed in the method according to the present invention is an amplification reaction of nucleic acids, which can be carried out in a quantitative manner such as for example the polymerase chain reaction. In a preferred embodiment of the present invention real time RT PCR may be used to quantify the level of marker mRNAs in samples of dysplasias or tumors (cells or tissue samples).

In another preferred embodiment of the invention the detection of the level of INK4a and/or proliferation marker gene-products is carried out by determining the level of expression of a protein or fragments thereof. The determination of the marker gene-product on the protein level can for example be carried out in a reaction comprising a binding agent specific for the detection of the particular marker polypeptide.

The binding agents can be used in many different detection techniques for example in western-blot, ELISA or immuno-precipitation. Generally polypeptide binding agent based detection can be carried out as well in vitro as directly in situ for example in the course of an immuno-chemical staining reaction. Any other method for determining the amount of particular polypeptides in biological samples can be used according to the present invention.

The immuno-cytochemical imaging procedures for use in the context of the present invention may comprise e.g. the staining of cytological or histological preparations with chromogenic or fluorescent dyes. The staining may e.g. comprise binding of the molecules to be detected by a first binding agent, which itself is detected by a secondary binding agent, which may be labelled. The first binding agent may in certain embodiments be a nucleic acid or a protein binding agent (e.g. an antibody) and the secondary binding agent may be e.g. a secondary antibody recognizing the first binding agent.

Any methods known in the art for performing staining of cytochemical or histochemical staining may be applied in the course of a method according to the present invention.

Binding agents as used in the context of the present invention for the detection of the level of INK4a polypeptides such as p16^{INK4a} or p14ARF polypeptides and proliferation marker polypeptides such as e.g. mcm5, mcm2, KI67, Ki-S5, PCNA or Ki-S2 polypeptides may comprise antibodies and antigen-binding fragments, bi-functional hybrid antibodies, peptidomimetics containing minimal antigen-binding epitopes, anti-cullines (anti-caline™) etc.

An antibody or antigen-binding agent is said to react specifically, if it reacts at a detectable level with a protein disclosed herein, and does not significantly react with other proteins. The antibodies which may be used in the present invention may be monoclonal or polyclonal antibodies. As used herein, the term antibody or monoclonal antibody is meant to include intact molecules as well as antibody fragments. Moreover, antibodies which may be used in the present invention include chimeric, single chain, and humanised antibodies.

According to the present invention binding agents may be used isolated or in combination. By means of combination it is possible to achieve a higher degree of sensitivity. The term antibody, preferably, relates to antibodies which consist essentially of pooled monoclonal antibodies with different epitopic specificities, as well as distinct monoclonal antibody preparations.

Monoclonal antibodies are made from antigen containing fragments of the polypeptide of the invention using any of a variety of techniques known to those of ordinary skill in the art; see, e.g., Harlow and Lane, Antibodies: A Laboratory Manual, Cold Spring Harbor Laboratory, 1988. In one such technique, an immunogen comprising the antigenic polypeptide or a synthetic part thereof is initially injected into any of a wide variety of mammals (e.g., mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Within the context of the present invention it must not necessarily be answered whether the one or the other proliferation marker is expressed in the cells. In certain embodiments main question may be whether any proliferation marker is expressed. Such in the course of the experiments a procedure was chosen, that gives the same fluorescence signal as indication of the presence of a proliferation marker. This procedure is suitable to improve sensitivity of the detection of the cell proliferation characteristics. As the case may be the procedure may be applied as to render one detectable signal for three, four or even more marker molecules being characteristic for cell proliferation. Analogously, the same may under certain circumstances be true for the INK4a gene expression products. it must be understood, that, as the case may be, different staining signals for different proliferation marker molecules may be desirable. The procedures may be applied to the necessities of the respective experiment.

The INK4a gene-products and/or proliferation marker gene products may according to the present invention be detected simultaneously. In this context simultaneously according to the present invention shall mean either literally at the same instant or within the same testing procedure, whereby the single detection steps are temporarily consecutive.

The detection procedure according to the present invention may furthermore comprise a cytochemical staining procedure rendering a chromogenic or fluorescent staining of cells or cell compartments. Such staining procedures are known to those of skill in the art and may for example comprise e.g. staining for acidophilic or basophilic structures, of subcellular regions (e.g. the nucleus, the mitochondria, the golgi, the cytoplasm etc.), of specific molecules (the chormosomes, of lipids, of glycoproteins, of polysaccharids etc.) in the cytological specimens. Fluorescence dyes such as DAPI, Quinacrin, Chromomycin, etc. may be employed. Furthermore chromogenic dyes such as Azan, Acridin-orange, Hematoxylin, Eosin, Sudan-red, Thiazin-stains (Toluidin-blue, Thionin) may be applied. In other embodiments staining procedures such as Pap-staining, Giemsa- staining, Hematoxylin-Eosin staining, van-Gieson staining, Schiff-staining (using Schiff reagent), staining procedures employing precipitation of metals (such as e.g. of silver in staining procedures employing Silver Nitrate) or insoluble stains such as e.g. of Turnbulls-blue (or other insoluble metal cyanides), etc. may be used in the course of a method as disclosed herein. It must be understood, that the named dyes and staining methods shall be examples for the applicable methods and that any other method known in the art may be applied to a method as disclosed herein.

The staining procedures may produce chromogenic stains for light microscopic inspection or fluorescent stains for inspection under fluorescence microscopic conditions. In another embodiment of the present invention radiation emitting procedures, procedures employing substances impairing the transmission of radiation or other contrast media for imaging of the cytological conditions in a sample (e.g. the generation of optical impression by means such as (micro-)autoradiographic or (micro-)radiographic picture generation) may be of use for a method according to the present invention.

All the staining and imaging procedures may be used for analysis not only in microscopic procedures but also in automated analysis procedures such flow cytometry, automated microscopic (computerized or computer aided) analysis or any other method for analysis of stained cytological specimens.

The analysis of the staining or imaging results of the different procedures may be performed in a single analysis step or in different subsequent steps. E.g. the light microscopic inspection of a specimen may be performed before or after fluorescence microsopic inspection of the specimen. In Fluorescence microscopy the analysis of different stains with different excitation wavelengths may be analyses simultaneous or subsequently. Other imaging methods may be employed simultaneously or subsequently to the named procedures.

There may be various circumstances, under which combinations of different staining methods will be suitable. E.g. in cases, where no satisfying cytological staining results may be achieved by immuno-chemical staining the additional application of general cytological staining techniques may be suitable.

A sample according to the method of the present invention may comprise any sample comprising cells. Samples may comprise e.g. secretions, smears, body fluids, and cell- or tissue-samples.

In one embodiment of the present invention samples comprise cells of the anogenital tract, of the respiratory tract or of the skin and its appendages. In certain embodiments the cells may be cells of the uterine cervix, the vagina, the vulva, the penis, the anus, the rectum, the bronchic tree, the lung, the peritoneum, the peritoneal space, the naso-pharyngeal space, the oral cavity or the skin. In certain embodiments of the present invention the sample may be a histological sample, a biopsy, or a cytological sample such as e.g. a smear, a swab, a wash, a body fluid containing cells (sputum, a secretion, saliva, etc.). In certain embodiments of the present invention the samples may comprise cells infected by papilloma virus. The samples may in certain embodiments comprise cervical smears, bronchioalveolar lavages etc.

In certain special embodiments of the present invention the sample may be prepared as a monolayer or thin layer preparation of a cytological specimen. The respective methods for preparation of monolayer or thin-layer preparation in cytology are known to those of skill in the art. In one embodiment the preparation may e.g. comprise the ThinPrep technology. Other methods comprise conventional smears, or method employing suspensions of cells for preparation of the cytological specimens.

Preparation of a sample may comprise e.g. obtaining a sample of a tissue, of a body fluid, of cells from a patient. According to the present invention preparation of the sample may also comprise several steps of further preparations of the sample, such as preparation of dissections, preparation of cell suspensions, spreading or applying the cells to be examined onto microscopic slides, preparation of tissue arrays, isolation of polypeptides or nucleic acids, preparation of solid phase fixed peptides or nucleic acids or preparation of beads, membranes or slides to which the molecules to be determined are coupled covalently or non-covalently.

In certain embodiments of the present invention the method may be performed in an automated manner. The automation of the method may be achieved by automated staining and analysis of histological or cytological specimens on a solid surface by microscopic means. In another embodiment the automation may comprise a flow-cytometric analysis of the staining of cells in solution.

The dysplastic lesions to which the method according to the present invention may be applied comprise any dysplastic lesions characterized by over-expression of INK4a gene products such as e.g. p16^{INK4a} or p14ARF. In certain embodiments those lesions are dysplasias associated with infections by papilloma viruses such as e.g. HPV. In one embodiment the HPV may be a high risk HPV subtype such as HPV16, HPV18, HPV31 , HPV 33, HPV35, HPV 39, HPV 45, HPV 51, HPV 52, HPV56, HPV 58, HPV 59, HPV 66, HPV 68 etc. In one embodiment the dysplastic lesions that may be detected according to the present invention comprise anogenital lesions, lesions of the respiratory tract, lesions of the head and the neck or lesions of the skin and its appendages. Such lesions may e.g. comprise dysplasias of the anus or rectum, of the vulva, the vagina, the cervix or the penis, of the bronchic tree, the lung, the oral cavity, or the nasopharyngeal space.

Another aspect of the present invention is a testing kit for performing the method according to the present invention. The kit may be for example a diagnostic kit or a research kit.

Thus a kit according to present invention comprises:
at least one or more probes for the detection of the presence or absence and/or the level of the over-expression of at least one INK4a gene-product and at least one cell proliferation marker gene product in biological samples, wherein the kit components are provided in a way to allow for simultaneous detection of at least one INK4a gene-product and at least one cell proliferation marker gene product in one single cell of specimens and wherein at least two distinguishable detectable signals are generated, at least one detectable signal being indicative for the level of expression of at least one INK4a gene product and at least one other detectable signal being indicative for the level of expression of at least one cell proliferation marker gene.

The reagents for the detection of the marker gene-products may include any agent capable of binding to the marker gene-products. Such reagents may include proteins, polypeptides, nucleic acids, peptide nucleic acids, glycoproteins, proteoglycans, polysaccharids or lipids.

The INK4a gene-product and proliferation marker gene product samples for canying out a positive control may comprise for example nucleic acids in applicable form, such as solution or salt, peptides in applicable form, tissue section samples or positive cells.

In a preferred embodiment of the invention the detection of the marker gene-products is carried out on the level of polypeptides. In this embodiment the binding agents may be for example antibodies specific for the marker gene-products or fragments thereof.

In another embodiment of the test kit the detection of the marker gene-products is carried out on the nucleic acid level. In this embodiment of the invention the reagent for the detection may be for example a nucleic acid probe or a primer reverse-complementary to said marker nucleic acids.

Thus the problem to be solved was to provide a method for discrimination between dysplastic cells and other cells lacking malignant growth potential. The method may be applied to any stage of dysplasias and may be especially useful in early stages, when cytological diagnostic methods based on the p16^{INK4a} over-expression needs a further information for the identification of metaplastic cells.

Furthermore the present invention provides a kit for performing the method according to the present invention.

### Brief description of the drawings

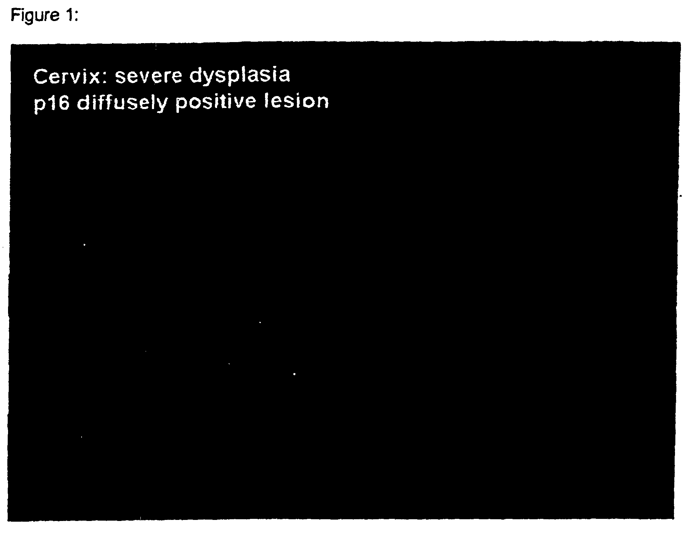

- Figure 1:: **Fluorescent staining of a histological specimen of the cervix uteri;** Figure 1 shows staining of a severe dysplasia using antibodies directed against p16^{INK4a}; for experimental details see Example 1; immunoreactivity for p16^{INK4a} renders green fluorescence. Almost all cells of the lesion are diffusely positive stained in the cytoplasm and in the nuclei.

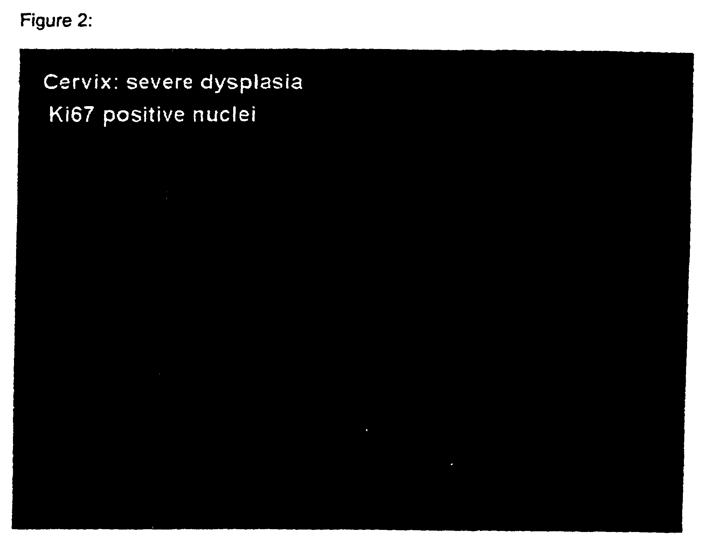

- Figure 2:: **Fluorescent staining of a histological specimen of the cervix uteri;** Figure 2 shows staining of a severe dysplasia using antibodies directed against Ki67; for experimental details see Example 1; immunoreactivity for Ki67 renders red fluorescence. Many cells of the dysplasia show nuclear positivity for Ki67.

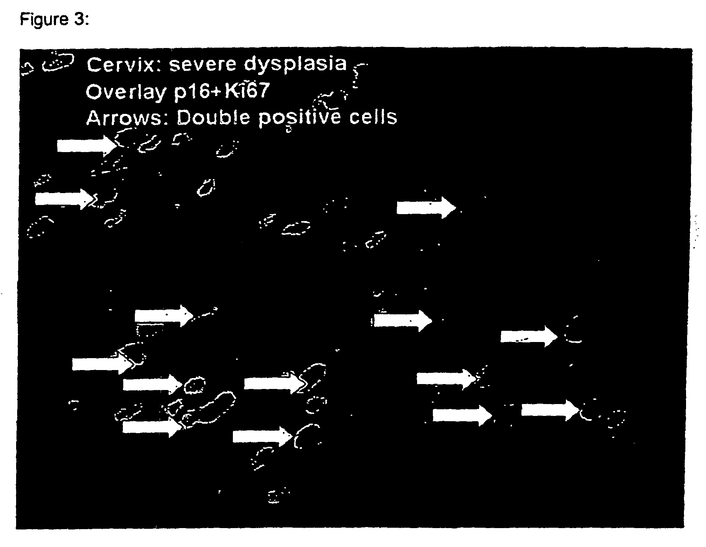

- Figure 3:: **Fluorescent double staining of a histological specimen of the cervix uteri;** Figure 3 shows staining of a severe dysplasia using antibodies directed against Ki67 and those directed against p16^{INK4a}; for experimental details see Example 1; immunoreactivity for Ki67 renders red fluorescence; immunoreactivity for p16^{INK4a} renders green fluorescence; overlay of red and green fluorescence renders yellow fluorescence. Many cells of the dysplasia show nuclear positivity for Ki67 as well as positivity for p 16^{INK4a} and give thus rise to yellow fluorescence.

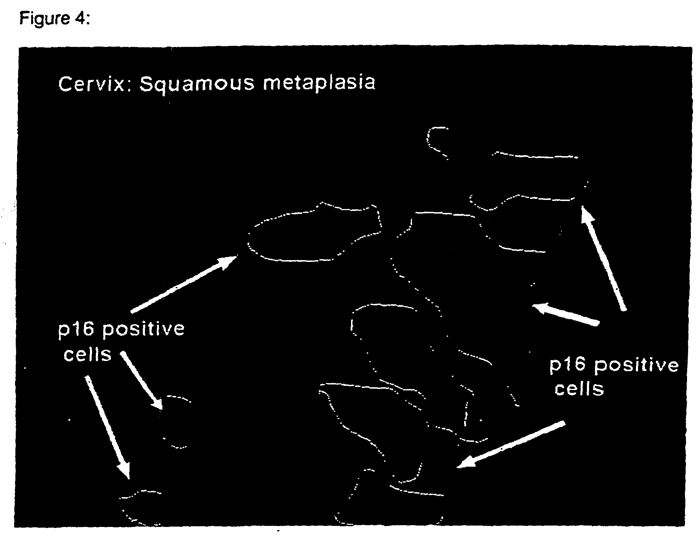

- Figure 4:: **Fluorescent staining of a histological specimen of the cervix uteri;** Figure 4 shows staining of a squamous metaplasia using antibodies directed against p16^{INK4a}; for experimental details see Example 1; immunoreactivity for p16^{INK4a} renders green fluorescence. Some cells of the metaplasia stain diffusely positive in the cytoplasm and in the nuclei.

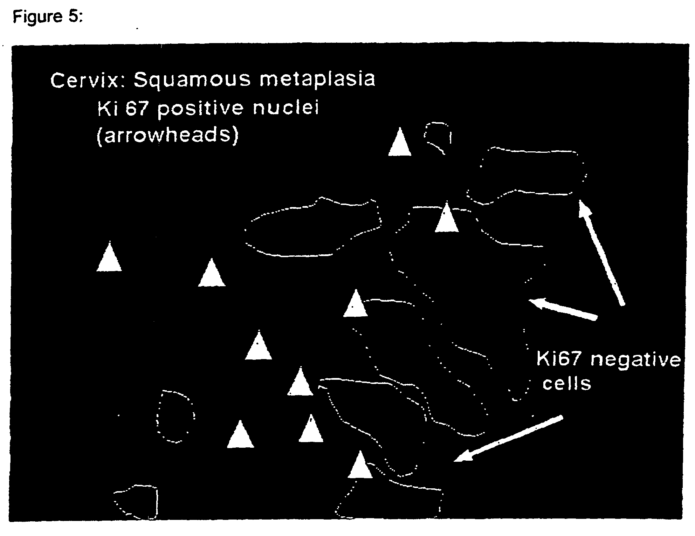

- Figure 5:: **Fluorescent staining of a histological specimen of the cervix uteri;** Figure 5 shows staining of a squamous metaplasia using antibodies directed against Ki67; for experimental details see Example 1; immunoreactivity for Ki67 renders red fluorescence. Many cells of the squamous metaplasia show nuclear positivity for Ki67. Areas, that have been identified as being positively expressing p16^{INK4a} do not show any positive staining for Ki67 indicating lack of expression of the antigen in these areas.

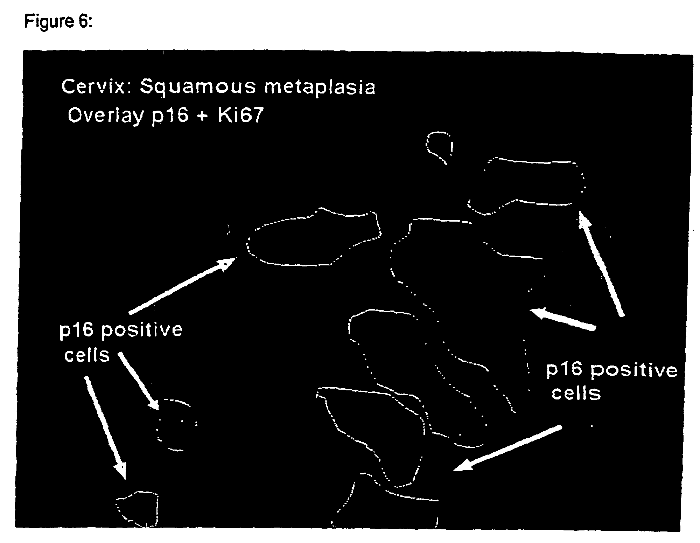

- Figure 6:: **Fluorescent double staining of a histological specimen of the cervix uteri;** Figure 6 shows staining of a severe dysplasia using antibodies directed against Ki67 and those directed against p16^{INK4a}; for experimental details see Example 1; immunoreactivity for Ki67 renders red fluorescence; immunoreactivity for p16^{INK4a} renders green fluorescence; overlay of red and green fluorescence renders yellow fluorescence. Areas, that are positively expressing p16^{INK4a} do not show any positive staining for Ki67 indicating lack of expression of the antigen in these areas. No cells in the specimen give rise to yellow fluorescence.

The following examples are given for the purpose of illustration only and are not intended to limit the scope of the invention disclosed herein. For the purpose of illustration the methods disclosed herein are exemplified using histological preparations. The histological examples aid to judge whether the cells stained in the one or the other way are to be classified as dysplastic or metaplastic. The methods may easily be transferred to cytological specimens by altering the protocol in appropriate manner. These alterations are known to those of ordinary skill in the art.

### Example 1: Immuno-fluorescent detection of the over-expression of p16^{INK4a} and Ki - 67 in samples of the uterine cervix ( double staining )

Sections of formalin fixed, paraffin embedded tissue samples of the cervix uteri were immuno-fluorescent stained using antibodies specific for p16^{INK4a} and Ki67.

The tissue sections were rehydrated through incubation in xylene and graded ethanol, and transferred to Aqua bidest. Antigen Retrieval was carried out with 10mM citrate buffer (pH 6.0) for p16^{INK4a} and Ki67 . Therefore the slides were heated in a waterbath for 40 min at 95°C - 98°C. The slides were cooled down to RT for 20 minutes, transferred to washing buffer (50mM Tris-HCl, 150mM NaCl, 0.05% Tween 20 / DakoCytomation: code no.: S3006).

To avoid non - specific binding of the secondary antibody (species : goat) the specimens were incubated with 10% goat serum for 30min at RT.

The slides were then incubated with the primary antibodies, mouse anti-human p16^{INK4a} antibody (3.48 µg/ml) and rabbit anti Ki67 (1:25) for 30 min at RT , the slides were then rinsed with washing buffer and placed in a fresh buffer bath for 5 min. Excess buffer was tapped off and each specimen was covered with 200µl of the secondary reagent , containing goat anti-mouse antibody , AlexaFluor®488 conjugated and goat anti rabbit antibody , Alexa Fluor®546 conjugated and then incubated for 30min at RT . Then slides were washed two times as before and directly mounted with a special mounting medium for fluorescence.

The microscopic examination of the slides reveals, that cells immuno-reactive with p16^{INK4a} and being also immuno-reactive for Ki67 may only be found in samples, that may microscopically be identified as samples of dysplastic lesions. Cells stained by the p16^{INK4a} specific reaction, that are originating from metaplasias, are not stained by the reaction specific for Ki67. The microscopic inspection of the cell proliferation marker staining shows, that metaplastic cells over-expressing p16^{INK4a} are not immuno-reactive with the antibodies directed against Ki67. Samples containing dysplastic tissue areas in contrast comprise cells, that are immuno-reactive with KI67 and with antibodies directed against p16^{INK4a}. So in contrast to dysplasias in metaplasias no cells may be double stained using the Ki67 and p16^{INK4a} specific antibodies.

Figures 1-6 show the staining results for a severe dysplasia of the cervix uteri and for a squamous metaplasia using antibodies directed against Ki67 and those directed against p16^{INK4a}. Immunoreactivity for Ki67 renders red fluorescence, immunoreactivity for p16^{INK4a} renders green fluorescence and overlay of red and green fluorescence renders yellow fluorescence. In the dysplastic specimen (Fig. 1-3) many cells of the dysplasia show nuclear positivity for Ki67 as well as positivity for p16INK4a and give thus rise to yellow fluorescence (see Figure 3) In contrast in the metaplastic specimen (Fig. 4-6) areas that are positively expressing p16^{INK4a} do not show any positive staining for Ki67 indicating lack of expression of the antigen in these areas. The double staining may be observed in this specimen (Fig. 6) and thus no cells in the specimen give rise to yellow fluorescence.

The results show, that the double staining of cells with reagents specific for Ki67 allows to discriminate p16^{INK4a} over-expressing metaplasias from dysplasias.

### Example 2: Detection of cells co-expressing p14ARF and mcm2 in samples of the uterine cervix by in situ hybridization

Smears of the uterine cervix may be semi-quantitatively analysed for the mRNA level of p16^{INK4a} and mcm2 in an in-situ staining reaction. The staining reaction is performed as follows:

For rehydration the spray-fixed smears are incubated in fresh 50% EtOH on a rocking device. The PEG film produced by the fixation procedure is removed by intensive rinsing. Following the smears are rinsed in aqua bidest. The smears are incubated with porteinase K (10µg/ml in PBS) for 10 min at 37°C. Then the slides are transferred to washing buffer (PBS / 0.1% Tween20) and finally the area containing the cells was surrounded with a lipid-pencil.

The hybridization mixture is prepared by mixing 50 µl of ready to use hybridization buffer (DAKO A/S, Glostrup, Danmark) with about 5 - 10 pmol of the probes. The probes are biotin-and Digoxygenin-labelled oligonucleotides of sequences complememtary to the respective mRNAs.

The hybridization mixture is heated to 95°C and afterwards equilibrated to 37°C. After the boiling procedure the smears are incubated with each 50 µl of the hybridization mixture for 4 hours at 42°C. The samples are washed in excess volumes of the wash buffers two times in 2 x SSC at 37°C for 15 min and once in 1 x SSC at 37 °C for 15 min Then the smears are rinsed two times at room temperature in 2 x SSC. Following this washing procedure the dissections are incubated for 30 min with blocking buffer (NEN, Blockingbuffer) at room temperature. Then follows 1 hour incubation with a 1:100 diluted (in Blocking buffer, see above) Strepavidine-alkaline phosphatase and with monoclonal mouse HRP-labeled anti-Digoxygenine antibodies (Molecular Probes). The smears are then washed 2 times in 1 x PBS/0,1% Triton X-100 for 10 min at room temperature, followed by one wash step with 1 x PBS, 50 mM MgCl₂ (pH 9,2) for 10 min at room temperature. Then the staining reaction is performed with ELF 97 phosphate (Molecular Probes) for 10 sec to 7 min at room temperature. Excess substrate is washed 3 times with 1x PBS/0,1% Triton X-100 for 10 min at room temperature. In a second staining step, the section is incubated with Tyramides-Alexa-Fluor 594 for 10 sec to 7 min. Excess substrate is washed 3 times with 1x PBS/0,1% Triton X-100 for 10 min at room temperature.. Finally the smears are dipped in H₂O_{dest.} and embedded with Fluorescence mounting medium (DakoCytomation). Then the stained dissections can be analysed by fluorescence microscopy.

The microscopic examination of the slides reveals, that cells positive for expression of p16^{INK4a} and also expressing mcm2 may only be found in samples, that may microscopically be identified as samples of dysplastic lesions. Cells stained by the p16^{INK4a} specific reaction, that are identifiable as metaplasias, are not stained by the reaction specific for mcm2. The microscopic inspection of the mRNA hybridisation shows, that metaplastic cells over-expressing p16^{INK4a} do not significantly express mRNA of mcm2. Dysplastic cells in contrast may be stained by in situ hybridisation with probes specific for mcm2 and with probes directed against p16^{INK4a}. So in contrast to dysplastic cells in metaplastic cells no double staining using the Ki67 and p16^{INK4a} specific probes may be observed.

The results show, that the double staining of cells with reagents specific for mcm2 allows to discriminate p16^{INK4a} over-expressing metaplasias from dysplasias.

### Example 3: Immuno-fluorescent detection of the over-expression of p16^{INK4a} and Ki-S2 in samples of the uterine cervix ( double staining)

Merckofix® fixed cytological samples (conventional smears and liquid based cytology (ThinPreps®)) of the cervix uteri were immuno-fluorescent stained using antibodies specific for p16^{INK4a} and Ki-S2.

Conventional smears and liquid based cytological samples (ThinPreps®) were rehydrated in ethanol (50%) for 10min and transferred in Aqua bidest. Antigen Retrieval was carried out with 10mM citrate buffer ( pH 6.0) for p16^{INK4a} and Ki67 . Therefore the slides were heated in a waterbath for 40 min at 95 °C - 98°C. The slides were cooled down to RT for 20 minutes, transferred to washing buffer (50mM Tris-HCl, 150mM NaCl, 0.05% Tween 20 / DakoCytomation: code no.: S3006) and finally the samples were surrounded with a lipid-pencil.

To avoid non - specific binding of the secondary antibody (species : goat) the specimens were incubated with 10% goat serum for 30min at RT.

The slides were then incubated with the primary antibodies, mouse anti-human p16^{INK4a} antibody (clone E6H4) (3.48 µg/ml) and rabbit anti Ki-S2 (1:25) for 30 min at RT , the slides were then rinsed with washing buffer and placed in a fresh buffer bath for 5 min. Excess buffer was tapped off and the specimen was covered with 200µl of the secondary reagent, containing goat anti-mouse antibody , AlexaFluor®488 conjugated and goat anti rabbit antibody , Alexa Fluor®546 conjugated and then incubated for 30min at RT . Then slides were washed two times as before and directly mounted with a special mounting medium for fluorescence.

The microscopic examination of the slides reveals, that cells immuno-reactive with p16^{INK4a} and being also immuno-reactive for Ki-S2 may microscopically be identified as dysplastic cells. Cells stained by the p16^{INK4a} specific reaction, which are not stained by the reaction specific for Ki-S2, may be classified by an experienced pathologist as either being metaplastic or being of endometrial origin. The microscopic inspection of the cell proliferation marker staining shows, that metaplastic cells over-expressing p16^{INK4a} are not immuno-reactive with the antibodies directed against Ki-S2. Dysplastic cells in contrast are immuno-reactive with Kl-52 and with antibodies directed against p16^{INK4a}. So in contrast to dysplastic cells, in metaplasias no cells may be double stained using the Ki-S2 and p16^{INK4a} specific antibodies.

The results show, that the double staining of cells with reagents specific for Ki-S2 allows to discriminate p16^{INK4a} over-expressing metaplastic cells from dysplastic cells.

### Example 4: Immuno-fluorescent detection of the over-expression of p16^{INK4a}, Ki67 and PCNA in bronchioalveolar-lavage samples of individuals with diagnosed small cell lung cancer (double staining)

Cells contained in bronchioalveolar lavage specimens of patients were prepared according to ThinPrep technology. Merckofix® fixed cytological samples of the lavages of patients with diagnosed small cell lung cancer were immuno-fluorescent stained using antibodies specific for p16^{INK4a}, Ki67and PCNA.

In this experiment a procedure was used, that does not discriminate between staining originating from immunoreactivity against the two proliferation markers PCNA and Ki67. Within the context of the present invention it must not necessarily be answered whether the one or the other proliferation marker is expressed in the cells. The main question is whether any proliferation marker is expressed.

Such in the course of the experiments a procedure was chosen, that gives the same fluorescence signal as indication of the presence of a proliferation marker. This procedure is suitable to improve sensitivity of the detection of the cell proliferation characteristics. As the case may be the procedure may be applied as to render one detectable signal for three four or even more marker molecules being characteristic for cell proliferation. Analogous the same may under certain circumstances be true for the INK4a gene expression products. It must be understood, that as the case may be different staining signals for different proliferation marker molecules may be desirable. The procedures may be applied to the necessities of the respective experiment.

The tissue sections were rehydrated through incubation in xylene and graded ethanol, and transferred to Aqua bidest. Conventional smears and liquid based cytological samples ( ThinPreps®) were rehydrated in ethanol (50%) for 10min and transferred in Aqua bidest. Antigen Retrieval was carried out with 10mM citrate buffer (pH 6.0) for p16^{INK4a}, Ki67and PCNA. Therefore the slides were heated in a waterbath for 40 min at 95 °C - 98°C. The slides were cooled down to RT for 20 minutes, transferred to washing buffer (50mM Tris-HCl, 150mM NaCl, 0.05% Tween 20 / DakoCytomation: code no.: S3006) and finally the samples were surrounded with a lipid-pencil.

To avoid non - specific binding of the secondary antibody (species : goat) the specimens were incubated with 10% goat serum for 30min at RT.

The slides were then incubated with the primary antibodies, mouse anti-human p16^{INK4a} antibody (3.48 µg/ml), rabbit anti Ki67 and rabbit anti PCNA (each 1:25) for 30 min at RT , the slides were then rinsed with washing buffer and placed in a fresh buffer bath for 5 min. Excess buffer was tapped off and the specimen was covered with 200µl of the secondary reagent , containing goat anti-mouse antibody , AlexaFluor®488 conjugated and goat anti rabbit antibody , Alexa Fluor®546 conjugated and then incubated for 30min at RT . Then slides were washed two times as before and directly mounted with a special mounting medium for fluorescence.

The microscopic examination of the slides reveals, that cells immuno-reactive with p16^{INK4a} and being also immuno-reactive for Ki67 or PCNA may microscopically be identified as cells of small cell lung cancer. Cells stained by the p16^{INK4a} specific reaction, that are originating from metaplasias, are not stained by the reaction specific for Ki67 and PCNA. The microscopic inspection of the cell proliferation marker staining shows, that metaplastic cells over-expressing p16^{INK4a} are not immuno-reactive with the antibodies directed against Ki67 and PCNA. Samples containing dysplastic cells in contrast comprise cells, that are immuno-reactive with Ki67 PCNA and with antibodies directed against p16^{INK4a}. So in contrast to dysplasias in metaplasias no cells may be triple stained using the Ki67 and PCNA and p16^{INK4a} specific antibodies.

The results show, that the triple staining of cells with reagents specific for Ki67/PCNA allows to discriminate p16^{INK4a} over-expressing non-dysplastic cells from dysplasias.

### Example 5: Flow cytometric detection of dysplastic cells by simultaneous detection of mcm5 mRNA, p14ARF protein and Ki67 protein in cells of cervical origin

Cytological samples (Cell suspensions in PBS, pH 7,4) of the cervix uteri were fluorescent stained using antibodies specific for p14ARF and Ki-67 and oligoprobes for mcm-5 and evaluated by three-color fluorescent FACS analysis.

Cells were centrifuged, the supernatant decanted and fixed and permeabilized with 100 ml Permeafix (Ortho Diagnostic, Raitan, NJ, USA) 1 hour at room temperature. Cells were washed in sterile PBS, pH 7,4, pelleted and resuspended in 100 ml Permeafix 1 hour at room temperature. Cells were washed in sterile PBS, pH 7,4, pelleted and resuspended in sterile PBS. They were incubated with PE-conjugated anti-p14ARF antibody and PE-Cy5-conjugated anti-Ki67 antibody for 1 h at +4°C. Cells were washed in sterile PBS, pH 7,4, pelleted and resuspended in 100 ml Permeafix 30 min at room temperature. Cells were washed in sterile PBS, pelleted by centrifugation, and then washed again in 2x standard saline citrate (SSC). After centrifugation , the cell pellet was resuspended in hybridisation solution (2x SSC, 30% formamide, sonicated salmon sperm, yeast transfer DNA) containing 500 ng of 5-carboxy-fluorescein double end labelled, mcm5-specific oligonucleotides probes. The intercellular hybridisation was performed at 42°C for 1 hour, followed by successive washes in 2x SSC, 0,5 % Triton X-100, and 1x SSC, 0,5 % Triton X-100 at 42°C. The cells were resuspended for analysis in PBS, pH 8,3 and analysed on a flow cytometer (FACScan, Becton Dickinson, IS). For each analysis 30.000-100.000 gated events were collected. Data analysis was performed using CellQuest (Becton Dickinson, IS)

The flow cytometer analysis reveals, that cells immuno-reactive with p14ARF and being also immuno-reactive for Ki-67 and/or reactive for the oligoprobes of mcm5 could be only identified in samples of patients with dysplastic lesions of the cervics. Samples from women with no dysplastic lesions showed no concomitant staining of p14ARF with Ki67 or mcm5. The results show, that the double or triple staining of cells with reagents specific for Ki67 and/or mcm5 allows to discriminate p14ARF over-expressing non-dysplastic cells from p14ARF over-expressing dysplastic cells.

## Claims

1. A method for discriminating dysplastic cells over-expressing INK4a gene products from other cells expressing INK4a gene products at a detectable level in biological samples comprising determining in a cytological or histological testing procedure the co-expression of at least two marker molecules in at least one single cell, wherein at least one marker molecule is an expression product encoded by the INK4a gene and at least one further marker molecule is a cell proliferation marker, wherein the over-expression of at least one INK4a gene product and expression of at least one marker for active cell proliferation at a detectable level within said single cell is indicative of the dysplastic state of the cell and wherein the over-expression of at least one INK4a gene product and expression of at least one marker for senescence, terminal cell differentiation, apoptosis or cell cycle arrest at a detectable level within said single cell is indicative of the non-dysplastic state of the cell.

2. A method according to claim 1, wherein a set of two or more cell proliferation markers is detected.

3. A method according to claim 1 or 2, wherein at least one INK4a gene-product has a molecular weight between 13 and 19 kDa.

4. A method according to claims 1 to 3, wherein at least one INK4a gene-product is selected from a group comprising p16^{INK4a} and p14ARF.

5. A method according to any one of the preceding claims, wherein at least one cell proliferation marker is selected from a group comprising a proliferation marker necessary for the maintenance of cell proliferation, a proliferation marker engaged in DNA replication, a proliferation marker being or encoding a member of the processive replication fork, a senescence marker, a cell cycle arrest marker and an apoptosis marker.

6. The method according to claim 5, wherein the gene-product necessary for the maintenance of cell proliferation is a molecule selected from a group comprising Ki67 molecules, Ki-S5 molecules and Ki-S2 molecules.

7. The method according to claim 5, wherein the gene-product engaged in DNA replication is selected from a group comprising helicases or subunits thereof, cell division cycle (cdc) molecules, phosphatase molecules and kinase molecules.

8. The method according to claim 7, wherein the helicases or subunits thereof are selected from a group comprising MCM2, MCM3, MCM4, MCM5, MCM6, MCM7 and HELAD1.

9. The method according to claim 7, wherein the cdc molecules, kinases and phosphatases are selected from a group comprising CDC6, CDC7 protein kinase, Dbf4, CDC14 protein phosphatase, CDC45 and MCM10.

10. The method according to claim 5, wherein the molecules engaged in the processive replication fork is selected from a group comprising PCNA and POLD.

11. A method according to any one of the claims 1-10, wherein the gene-product is a polypeptide or a nucleic acid molecule.

12. A method according to any one of the preceding claims, wherein additionally at least one further marker molecule is detected for improvement of the assessment of diagnosis or prognosis.

13. A method according to claim 12, wherein the further marker molecule is at least one further proliferation marker molecule.

14. A method according to claim 12 or 13, wherein the further marker molecule is selected from a group comprising a senescence marker, an apoptosis marker, a cell cycle arrest marker, a marker for terminal differentiation of cells, a marker for viral infection, a marker for viral activity, a cell cycle regulatory protein, a gene-product necessary for the maintenance of cell proliferation, a gene-product engaged in DNA replication, a gene-product being a member of the processive replication fork.

15. A method according to any one of the preceding claims, wherein additionally a cytological staining procedure employing at least one dye selected from a group comprising DAPI, Quinacrin, Chromomycin, Azan, Acridin-orange, Hematoxylin, Eosin, Sudan-red, Toluidin-blue, and Thionin, or a staining method selected from a group comprising Pap-staining, Giemsa-staining, Hematoxylin-Eosin staining, van-Gieson-staining, Schiff-staining, staining via metal precipitates, Turnbulls-blue-staining and staining via metal cyanides is applied.

16. The method according to any one of the preceding claims, wherein the dysplastic cells are cells of a cancerous or precancerous lesion.

17. The method according to claim 16, wherein the dysplastic cells are cells of a dysplasia being associated with a papilloma virus.

18. The method according to claim 17, wherein the papilloma virus is high risk human papilloma virus selected from a group comprising HPV16, HPV18, HPV31, HPV 33, HPV35, HPV 39 HPV 45, HPV 51, HPV 52, HPV56, HPV 58, HPV 59, HPV 66 and HPV 68.

19. The method according to any one of the claims 16 to 18, wherein the lesion is selected from a group comprising a lesion of the anogenital tract, a lesion of the respiratory tract and a lesion of the skin and it's appendages.

20. The method according to claim 17 or 19, wherein the lesion is selected from a group comprising a lesion of the uterine cervix, a lesion of the vagina, a lesion of the vulva, a lesion of the penis, a lesion of the anus, a lesion of the rectum, a lesion of the bronchic tree, a lesion of the lung, a lesion of the peritoneal space, a lesion of the naso-pharyngeal space, a lesion of the oral cavity or a lesion of the skin.

21. A method according to any preceding claim, wherein the biological sample is a sample containing cells originating from the anogenital tract, from the respiratory tract or from the skin and its appendages .

22. A method according to claim 21, wherein the cells are cells originating from the uterine cervix, the vagina, the vulva, the penis, the anus, the rectum, the bronchic tree, the lung, the naso-pharyngeal space, the oral cavity or the skin.

23. A method according to any one of the preceding claims, wherein the biological sample is a cytological or histological preparation.

24. A method according to any one of the preceding claims, wherein the detection of the INK4a gene-products and/or the cell proliferation marker molecules is performed using at least one probe specifically recognising at least one of the respective molecules to be detected.

25. A method according to claim 24, wherein at least one probe is detectably labelled.

26. A method according to claim 25, wherein at least one label is selected from the group consisting of a radioisotope, a bioluminescent compound, a chemiluminescent compound, a fluorescent compound, a metal chelate, or an enzyme.

27. A method according to any one of the claims 24 to 26, wherein at least one probe is a protein and/or a nucleic acid.

28. A method according to claim 27, wherein at least one probe is an antibody directed against a INK4a encoded gene-product or a cell proliferation marker gene product.

29. The method according to claim 28, which comprises an immuno-cytochemical staining procedure.

30. The method according to claim 25 or 26, wherein at least one probe is a nucleic acid specifically hybridizing to an INK4a gene-product or a cell proliferation marker gene product.

31. The method according to claim 30, which comprises an in situ hybridization reaction.

32. The method according to claim 30, which comprises a nucleic acid amplification reaction.

33. The method according to claim 32, wherein the nucleic acid amplification reaction is PCR, NASBA or LCR.

34. A method according to any of the preceding claims, wherein detection reactions using nucleic acid probes and polypeptide probes are carried out simultaneously.

35. A kit for performing a method according to any one of the preceding Claims, which is a diagnostic kit or a research kit, comprising at least one or more probes for the detection of the presence or absence and/or the level of the over-expression of at least one INK4a gene-product and at least one cell proliferation marker gene product in biological samples, wherein the kit components are provided in a way to allow for simultaneous detection of at least one INK4a gene-product and at least one cell proliferation marker gene product in one single cell of specimens and wherein at least two distinguishable detectable signals are generated, at least one detectable signal being indicative for the level of expression of at least one INK4a gene product and at least one other detectable signal being indicative for the level of expression of at least one cell proliferation marker gene.

36. A kit according to claim 35, wherein the INK4a gene products are selected from a group comprising p16^{INK4a} and p14ARF.

37. A kit according to claim 35 or 36, wherein the cell proliferation marker gene products are selected from a group comprising CDC6, MCM3, MCM3, MCM4, MCM5, MCM6, MCM7, CDC7 protein kinase, Dbf4, CDC14 protein phosphatase, CDC45 and MCM10, Ki67, Ki-S2, PCNA or POLD.

38. The kit according to claims 35 to 37 furthermore comprising at least one of the following
a. a p16^{INK4a} sample for carrying out a positive control reaction
b. a p14ARF sample for carrying out a positive control reaction
c. a Ki67 sample for carrying out a positive control reaction
d. a Ki-S2 sample for carrying out a positive control reaction
e. an MCM5 sample for carrying out a positive control reaction
f. an MCM2 sample for carrying out a positive control reaction
g. a PCNA sample for carrying out a positive control reaction
h.reagents for detection of the presence or absence and/or the level of p16INK4a
i.reagents for detection of the presence or absence and/or the level of p14ARF
j. reagents for detection of the presence or absence and/or the level of Ki67
k. reagents for detection of the presence or absence and/or the level of Ki-S2
l. reagents for detection of the presence or absence and/or the level of MCM5
m. reagents for detection of the presence or absence and/or the level of MCM2
n. reagents for detection of the presence or absence and/or the level of PCNA
o. one or more samples of INK4a gene-products for carrying out positive control reactions
p. one or more samples of cell proliferation marker gene-products for carrying out positive control reactions
q. one or more reagents for the detection of the presence or absence and/or the level of other INK4a gene products
r. and one or more reagents for the detection of the presence or absence and/or the level of other cell proliferation marker gene products.

## Patentansprüche

1. Verfahren zur Unterscheidung dysplastischer Zellen, die INK4a Genprodukte überexprimieren, von anderen Zellen, die INK4a Genprodukte in einem nachweisbaren Level exprimieren, in biologischen Proben, umfassend die Bestimmung der Co-Expression von mindestens zwei Markermolekülen in mindestens einer einzelnen Zelle in einem zytologischen oder histologischen Testverfahren, wobei mindestens ein Markermolekül ein Expressionsprodukt ist, das durch INK4a Gene kodiert wird, und mindestens ein weiteres Markermolekül ein Zellproliferationsmarker ist, wobei die Überexpression von mindestens einem INK4a Genprodukt und die Expression von mindestens einem Marker für aktive Zellproliferation in einem nachweisbaren Level innerhalb besagter einzelner Zelle hinweisend für den dysplastischen Zustand der Zelle ist, und wobei die Überexpression von mindestens einem INK4a Genprodukt und die Expression von mindestens einem Marker für Seneszenz, terminale Zelldifferenzierung, Apoptose oder Zellzyklusarrest in einem nachweisbaren Level innerhalb besagter einzelner Zelle hinweisend für den nicht-dysplastischen Zustand der Zelle ist.

2. Verfahren gemäß Anspruch 1, wobei ein Set von zwei oder mehreren Zellproliferationsmarkern nachgewiesen wird.

3. Verfahren gemäß Anspruch 1 oder 2, wobei mindestens ein INK4a Genprodukt ein molekulares Gewicht zwischen 13 und 19 kDa hat.

4. Verfahren gemäß der Ansprüche 1 bis 3, wobei mindestens ein INK4a Genprodukt aus einer Gruppe umfassend p16^{INK4a} und p14ARF ausgewählt ist.

5. Verfahren gemäß einem beliebigen der vorgehenden Ansprüche, wobei mindestens ein Zellproliferationsmarker aus einer Gruppe umfassend für die Aufrechterhaltung der Zellproliferation erforderliche Proliferationsmarker, an der DNA Replikation beteiligte Proliferationsmarker, Proliferationsmarker, die ein Mitglied der prozessiven Replikationsgabel sind oder diese kodieren, Seneszenzmarker, Zellzyklusarrestmarker und Apoptosemarker, ausgewählt ist.

6. Verfahren gemäß Anspruch 5, wobei das Genprodukt, das für die Aufrechterhaltung der Zellproliferation erforderlich ist, ein Molekül ist, ausgewählt aus einer Gruppe umfassend Ki67 Moleküle, Ki-S5 Moleküle und Ki-S2 Moleküle.

7. Verfahren gemäß Anspruch 5, wobei das Genprodukt, das an der DNA Replikation beteiligt ist, ausgewählt ist aus einer Gruppe umfassend Helikasen oder Untereinheiten davon, Zellteilungszyklusmoleküle (cdc), Phosphatasemoleküle und Kinasemoleküle.

8. Verfahren gemäß Anspruch 7, wobei die Helikasen oder Untereinheiten davon ausgewählt sind aus einer Gruppe enthaltend MCM2, MCM3; MCM4, MCM5, MCM6, MCM7 und HELAD1.

9. Verfahren gemäß Anspruch 7, wobei die cdc Moleküle, Kinasen und Phosphatasen ausgewählt sind aus einer Gruppe enthaltend CDC6, CDC7 Protein Kinase, Dbf4, CDC14 Proteinphosphatase, CDC45 und MCM10.

10. Verfahren gemäß Anspruch 5, wobei die Moleküle, die an der prozessiven Replikationsgabel beteiligt sind, aus einer Gruppe umfassend PCNA und POLD ausgewählt sind.

11. Verfahren gemäß einem beliebigen der Ansprüche 1-10, wobei das Genprodukt ein Polypeptid oder ein Nukleinsäuremolekül ist.

12. Verfahren gemäß einem beliebigen der vorgehenden Ansprüche, wobei zusätzlich mindestens ein weiteres Markermolekül zur Verbesserung der Diagnose- oder Prognosestellung nachgewiesen wird.

13. Verfahren gemäß Anspruch 12, wobei das weitere Markermolekül mindestens ein weiteres Proliferationsmarkermolekül ist.

14. Verfahren gemäß Anspruch 12 oder 13, wobei das weitere Markermolekül ausgewählt ist aus einer Gruppe umfassend Seneszenzmarker, Apoptosemarker, Zellzyklusarrestmarker, Marker für terminale Zelldifferenzierung, Marker für virale Infektion, Marker für virale Aktivität, zellzyklusregulierende Proteine, für die Aufrechterhaltung von Zellproliferation erforderliche Genprodukte, an der DNA Replikation beteiligte Genprodukte, Genprodukte, die ein Teil der prozessiven Replikationsgabel sind.

15. Verfahren gemäß einem beliebigen der vorgehenden Ansprüche, wobei zusätzlich ein zytologisches Färbeverfahren unter Einsatz mindestens eines Farbstoffs ausgewählt aus einer Gruppe umfassend DAPI, Quinacrin, Chromomyzin, Azan, Acridin-Orange, Hematoxylin, Eosin, Sudan-Rot, Toluidin-Blau, und Thionin, oder eine Färbemethode ausgewählt aus einer Gruppe umfassend Pap-Färbung, Giemsa-Färbung, Hematoxylin-Eosin-Färbung, van-Gieson-Färbung, Schiff-Färbung, Färbung durch Metallpräzipitate, Tumbulls-Blau-Färbung und Färbung durch Metallzyanide angewendet wird.

16. Verfahren gemäß einem beliebigen der vorgehenden Ansprüche, wobei die dysplastischen Zellen Krebszellen oder Zellen einer Krebsvorstufe sind.

17. Verfahren gemäß Anspruch 16, wobei die dysplastischen Zellen Zellen einer Papillomvirus assoziierten Dysplasie sind.

18. Verfahren gemäß Anspruch 17, wobei das Papillomvirus ein Hochrisiko-HPV, ausgewählt aus einer Gruppe umfassend HPV16, HPV18, HPV31, HPV33, HPV35, HPV39, HPV45, HPV51, HPV52, HPV56, HPV58, HPV59, HPV66 und HPV68 ist.

19. Verfahren gemäß einem beliebigen der Ansprüche 16 bis 18, wobei die Läsion aus einer Gruppe umfassend Läsionen des Anogenitaltraktes, Läsionen des Respirationstraktes und Läsionen der Haut und deren Anhangsgebilde ausgewählt ist.

20. Verfahren gemäß Anspruch 17 oder 19, wobei die Läsion aus einer Gruppe umfassend die Läsionen des Gebärmutterhalses, Läsionen der Vagina, Läsionen der Vulva, Läsionen des Penis, Läsionen des Anus, Läsionen des Rektum, Läsionen des Bronchialbaums, Läsionen der Lunge, Läsionen des Bauchfellraums, Läsionen des Hals-Nasenraums, Läsionen der Mundhöhle oder Läsionen der Haut ausgewählt wird.

21. Verfahren gemäß einem beliebigen vorgehenden Anspruch, wobei die biologische Probe eine Probe ist, die Zellen enthält, welche vom Anogenitaltrakt, vom Respirationstrakt oder von der Haut und deren Anhangsgebilden stammen.

22. Verfahren gemäß Anspruch 21, wobei die Zellen Zellen sind, die vom Gebärmutterhals, von der Vagina, der Vulva, dem Penis, dem Anus, dem Rektum, dem Bronchialbaum, der Lunge, dem Hals-Nasenraum, der Mundhöhle oder der Haut stammen.

23. Verfahren gemäß einem beliebigen der vorgehenden Ansprüche, wobei die biologische Probe ein zytologisches oder histologisches Präparat ist.

24. Verfahren gemäß einem beliebigen der vorgehenden Ansprüche, wobei der Nachweis der INK4a Genprodukte und/oder der Zellproliferationsmarkermoleküle mit mindestens einer Sonde durchgeführt wird, die mindestens eines der jeweiligen nachzuweisenden Moleküle erkennt.

25. Verfahren gemäß Anspruch 24, wobei mindestens eine Sonde eine nachweisbare Markierung trägt.

26. Verfahren gemäß Anspruch 25, wobei mindestens eine Markierung aus der Gruppe ausgewählt ist, die aus Radioisotopen, biolumineszenten Verbindungen, chemilumineszenten Verbindungen, fluoreszenten Verbindungen, Metallchelaten, oder Enzymen besteht.

27. Verfahren gemäß einem beliebigen der Ansprüche 24 bis 26, wobei mindestens eine Sonde ein Protein und/oder eine Nukleinsäure ist.

28. Verfahren gemäß Anspruch 27, wobei mindestens eine Sonde ein Antikörper ist, der gegen ein INK4a kodiertes Genprodukt oder ein Zellproliferationsmarker-Genprodukt gerichtet ist.

29. Verfahren gemäß Anspruch 28, welches ein immunozytochemisches Färbeverfahren umfasst.

30. Verfahren gemäß der Ansprüche 25 oder 26, wobei mindestens eine Sonde eine Nukleinsäure ist, die spezifisch an ein INK4a-Genprodukt oder ein Zellproliferationsmarker-Genprodukt hybridisiert.

31. Verfahren gemäß Anspruch 30, welches eine In-Situ Hybridisierungsreaktion umfasst.

32. Verfahren gemäß Anspruch 30, welches eine Nukleinsäureamplifikationsreaktion umfasst.

33. Verfahren gemäß Anspruch 32, wobei die Nukleinsäureamplifikationsreaktion PCR, NASBA oder LCR ist.

34. Verfahren gemäß einem beliebigen der vorgehenden Ansprüche, wobei Nachweisreaktionen mit Nukleinsäuresonden und Polypeptidsonden gleichzeitig durchgeführt werden.

35. Ein Kit zur Durchführung eines Verfahrens gemäß einem beliebigen der vorgehenden Ansprüche, welches ein diagnostisches Kit oder ein Forschungskit ist, umfassend mindestens eine oder mehrere Sonden zum Nachweis der Anwesenheit oder Abwesenheit und/oder des Levels der Überexpression von mindestens einem INK4a Genprodukt und mindestens einem Zellproliferationsmarker-Genprodukt in biologischen Proben, wobei die Kitkomponenten so zur Verfügung gestellt werden, dass ein gleichzeitiger Nachweis von mindestens einem INK4a Genprodukt und mindestens einem Zellproliferationsmarker-Genprodukt in einer einzigen Zelle der Proben möglich ist, und wobei mindestens zwei unterscheidbare nachweisbare Signale erzeugt werden, mindestens ein nachweisbares Signal hinweisend für den Level der Expression von mindestens einem INK4a Genprodukt ist, und mindestens ein anderes nachweisbares Signal hinweisend für den Level der Expression von mindestens einem Zellproliferationsmarkergen ist.

36. Ein Kit gemäß Anspruch 35, wobei die INK4a Genprodukte aus einer Gruppe umfassend p16^{INK4a} und p14ARF ausgewählt sind.

37. Ein Kit gemäß der Ansprüche 35 oder 36, wobei die Zellproliferationsmarker-Genprodukte ausgewählt sind aus einer Gruppe enthaltend CDC6, MCM3, MCM3, MCM4, MCM5, MCM6, MCM7, CDC7 Proteinkinase, Dbf4, CDC14 Proteinphosphatase, CDC45 und MCM10, Ki67, Ki-S2, PCNA oder POLD.

38. Das Kit gemäß der Ansprüche 35 bis 37 weiterhin umfassend mindestens Eins der Folgenden:
a. eine p16^{INK4a} Probe zur Durchführung einer positiven Kontrollreaktion
b. eine p14ARF Probe zur Durchführung einer positiven Kontrollreaktion
c. eine Ki67 Probe zur Durchführung einer positiven Kontrollreaktion
d. eine Ki-S2 Probe zur Durchführung einer positiven Kontrollreaktion
e. eine MCM5 Probe zur Durchführung einer positiven Kontrollreaktion
f. eine MCM2 Probe zur Durchführung einer positiven Kontrollreaktion
g. eine PCNA Probe zur Durchführung einer positiven Kontrollreaktion
h. Reagenzien zum Nachweis der Anwesenheit oder Abwesenheit und/oder des Levels von p16INK4a
i. Reagenzien zum Nachweis der Anwesenheit oder Abwesenheit und/oder des Levels von p14ARF
j. Reagenzien zum Nachweis der Anwesenheit oder Abwesenheit und/oder des Levels von Ki67
k. Reagenzien zum Nachweis der Anwesenheit oder Abwesenheit und/oder des Levels von Ki-S2
l. Reagenzien zum Nachweis der Anwesenheit oder Abwesenheit und/oder des Levels von MCM5
m. Reagenzien zum Nachweis der Anwesenheit oder Abwesenheit und/oder des Levels von MCM2
n. Reagenzien zum Nachweis der Anwesenheit oder Abwesenheit und/oder des Levels von PCNA
o. eine oder mehrere Proben von INK4a Genprodukten zur Durchführung positiver Kontrollreaktionen
p. eine oder mehrere Proben von Zellproliferationsmarker-Genprodukten zur Durchführung positiver Kontrollreaktionen
q. eine oder mehrere Reagenzien zum Nachweis der Anwesenheit oder Abwesenheit und/oder des Levels anderer INK4a Genprodukte
r. und eine oder mehrere Reagenzien zum Nachweis der Anwesenheit oder Abwesenheit und/oder des Levels anderer Zellproliferationsmarker-Genprodukte.

## Revendications

1. Procédé permettant de discriminer des cellules dysplasiques surexprimant des produits du gène INK4a d'autres cellules exprimant des produits du gène INK4a à un niveau détectable, dans des échantillons biologiques comprenant la détermination de la coexpression d'au moins deux molécules marqueurs dans au moins une seule cellule par un procédé de test cytologique ou histologique, **caractérisé en ce qu'**au moins un molécule marqueur est un produit d'expression codé par le gène INK4a et **en ce qu'**au moins une autre molécule marqueur est un marqueur de prolifération cellulaire, **caractérisé en ce que** la surexpression d'au moins un produit du gène INK4a et l'expression d'au moins un marqueur pour la prolifération cellulaire active à un niveau détectable dans ladite seule cellule, est indicative pour l'état dysplasique de la cellule, et **caractérisé en ce que** la surexpression d'au moins un produit du gène INK4a et l'expression d'au moins un marqueur pour la sénescence, la différenciation terminale de la cellule, l'apoptose ou l'inhibition du cycle cellulaire à un niveau détectable dans ladite seule cellule, est indicative pour l'état non dysplasique de la cellule.

2. Procédé selon la revendication 1, **caractérisé en ce qu'**un ensemble de deux ou plusieurs marqueurs de prolifération cellulaire est détecté.

3. Procédé selon la revendication 1 ou 2, **caractérisé en ce qu'**au moins un produit du gène INK4a a un poids moléculaire compris entre 13 et 19 kDa.

4. Procédé selon les revendications 1 à 3, **caractérisé en ce qu'**au moins un produit du gène INK4a est sélectionné dans un groupe comprenant p16^{INK4a} et p14ARF.

5. Procédé selon l'une quelconque des revendications précédentes , **caractérisé en ce qu'**au moins un marqueur de prolifération cellulaire est sélectionné dans un groupe comprenant des marqueurs de prolifération nécessaires au maintien de la prolifération cellulaire, des marqueurs de prolifération occupés à la réplication de l'ADN, des marqueurs de prolifération étant ou codant un membre de la fourche de réplication processive, des marqueurs de sénescence, des marqueurs de l'inhibition du cycle cellulaire ou des marqueurs de l' apoptose.

6. Procédé selon la revendication 5, **caractérisé en ce que** le produit du gène nécessaire au maintien de la prolifération cellulaire est une molécule sélectionnée dans un groupe comprenant des molécules Ki-67, Ki-S5, et Ki-S2.

7. Procédé selon la revendication 5, **caractérisé en ce que** le produit du gène occupé à la réplication de l'ADN est sélectionné dans un groupe comprenant des hélicases ou les sous-unités des hélicases, des molécules du cycle de division cellulaire (cdc), des molécules phosphatase et kinase.

8. Procédé selon la revendication 7, **caractérisé en ce que** des hélicases ou des sous-unités d'hélicases sont sélectionnées dans un groupe comprenant MCM2, MCM3, MCM4, MCM5, MCM6, MCM7, et HELAD1.

9. Procédé selon la revendication 7, **caractérisé en ce que** les molécules cdc, kinases et phosphatases sont sélectionnées dans un groupe comprenant CDC6, CDC7 protéine kinase, Dbf4, CDC14 protéine phosphatase, CDC45 et MCM10.

10. Procédé selon la revendication 5, **caractérisé en ce que** les molécules occupées à la fourche de réplication processive sont sélectionnées dans un groupe comprenant PCNA et POLD.

11. Procédé selon l'une quelconque des revendications 1 à 10, **caractérisé en ce que** le produit du gène est un polypeptide ou une molécule d'acide nucléique.

12. Procédé selon l'une quelconque des revendications précédentes, **caractérisé en ce qu'**en outre au moins une autre molécule marqueur est détectée pour l'amélioration du diagnostic ou pronostic.

13. Procédé selon la revendication 12, **caractérisé en ce que** l'autre molécule marqueur est au moins un autre marqueur de prolifération moléculaire.

14. Procédé selon la revendication 12 ou 13, **caractérisé en ce que** l'autre molécule marqueur est sélectionné dans un groupe comprenant des marqueurs de sénescence, des marqueurs de l'apoptose, des marqueurs de l'inhibition du cycle cellulaire, des marqueurs pour la différentiation terminale des cellules, des marqueurs pour l'infection virale, des marqueurs pour l'activité virale, des protéines régulant le cycle cellulaire, des produits du gène nécessaires au maintien de la prolifération cellulaire, des produits du gène occupés à la réplication de l'ADN, des produits du gène étant membre de la fourche de réplication processive.

15. Procédé selon l'une quelconque des revendications précédentes, **caractérisé en ce qu'**en outre s'applique un procédé de coloration cytologique en employant au moins un colorant sélectionné dans un groupe comprenant DAPI, Quinacrin, Chromomycin, Azan, Acridin-orange, Hematoxylin, Eosin, Sudan-rouge, Toluidin-bleu, et Thionin, ou un procédé de coloration sélectionné dans un groupe comprenant la coloration Pap, la coloration Giemsa, la coloration Hematoxylin-Eosin, la coloration van-Gieson, la coloration Schiff, la coloration par précipitats de métal, la coloration Tumbulls-bleu, et la coloration par cyanides de métal.

16. Procédé selon l'une quelconque des revendications précédentes, **caractérisé en ce que** les cellules dysplasiques sont des cellules d'une lésion cancéreuse ou pré-cancéreuse.

17. Procédé selon la revendication 16, **caractérisé en ce que** les cellules dysplasiques sont des cellules d'une dysplasie associée au papilloma virus.

18. Procédé selon la revendication 17, **caractérisé en ce que** le papilloma virus et un papilloma virus humain à haut risque sélectionné dans un groupe comprenant HPV16, HPV18, HPV31, HPV33, HPV35, HPV39, HPV45, HPV51, HPV52, HPV56, HPV58, HPV59, HPV66 et HPV68.

19. Procédé selon l'une quelconque des revendications 16 à 18, **caractérisé en ce que** la lésion est sélectionnée dans un groupe comprenant des lésions de l'appareil ano-génital, des lésions de l'appareil respiratoire et des lésions de la peau et des phanères de la peau.

20. Procédé selon la revendication 17 ou 19 **caractérisé en ce que** la lésion est sélectionnée dans un groupe comprenant des lésions du col de l'utérus, des lésions du vagin, des lésions de la vulve, des lésions du pénis, des lésions de l'anus, des lésions du rectum, des lésions de l'arbre bronchial, des lésions du poumon, des lésions de l'espace péritonéique, des lésions de l'espace nasopharyngeal, des lésions de la cavité buccale ou des lésions de la peau.

21. Procédé selon l'une quelconque des revendications précédentes, **caractérisé en ce que** l'échantillon biologique est une échantillon contenant des cellules provenant de l'appareil ano-génital, de l'appareil respiratoire ou de la peau et des phanères de la peau.

22. Procédé selon la revendication 21, **caractérisé en ce que** les cellules sont des cellules provenant du col de l'utérus , du vagin, de la vulve, du pénis, de l'anus, du rectum, de l'arbre bronchial, du poumon, de l'espace péritonéal de la cavité buccale ou de la peau.

23. Procédé selon l'une quelconque des revendications précédentes, **caractérisé en ce que** l'échantillon est une préparation cytologique ou histologique.

24. Procédé selon l'une quelconque des revendications précédentes, **caractérisé en ce que** la détection des produits du gène INK4a et/ou des molécules du marqueur de prolifération cellulaire est réalisée à l'aide d'au moins une sonde qui reconnaît spécifiquement au moins une des molécules respectives à détecter.

25. Procédé selon la revendication 24, **caractérisé en ce qu'**au moins une sonde est marquée de manière détectable.

26. Procédé selon la revendication 25, **caractérisé en ce qu'**au moins un marqueur détectable est choisi dans un groupe comprenant des isotopes radioactifs, des composés bioluminescents, des composés chémoluminescents, des composés fluorescents, des chélates de métal ou des enzymes.

27. Procédé selon l'une quelconque des revendications 24 à 26, **caractérisé en ce qu'**au moins une sonde est une protéine et/ou un acide nucléique.

28. Procédé selon la revendication 27, **caractérisé en ce qu'**au moins une sonde est un anticorps dirigé contre un produit du gène codant INK4a ou un produit du gène du marqueur de prolifération cellulaire.

29. Procédé selon la revendication 28 comprenant un procédé de coloration immuno-cytochimique.

30. Procédé selon la revendication 25 ou 26, **caractérisé en ce qu'** au moins une sonde est un acide nucléique s'hybridant spécifiquement à un produit du gène INK4a ou un produit du gène du marqueur de prolifération cellulaire.

31. Procédé selon la revendication 30, comprenant une réaction d' hybridation in situ.

32. Procédé selon la revendication 30, comprenant une réaction d'amplification des acides nucléiques.

33. Procédé selon la revendication 32, **caractérisé en ce que** la réaction d'amplification des acides nucléiques est PCR, NASBA ou LCR.

34. Procédé selon l'une quelconque des revendications précédentes, **caractérisé en ce que** les réactions de détection avec des sondes d'acides nucléiques et des polypeptides sont réalisées simultanément.

35. Kit pour la mise en oeuvre du procédé selon l'une quelconque des revendications précédentes, étant un kit de diagnostic ou de recherche, comprenant au moins une ou plusieurs sondes pour la détection de la présence, de l'absence et/ou de la détermination du niveau de surexpression d'au moins un produit du gène INK4a et d'au moins un produit du gène du marqueur de prolifération cellulaire dans des échantillons biologiques, **caractérisés en ce que** les composants du kit sont fournis de manière à permettre une détection simultanée d'au moins un produit du gène INK4a et d'au moins un produit du gène du marqueur de la prolifération cellulaire dans une seule cellule des échantillons et **caractérisé en ce qu'**au moins deux signaux distincts détectables sont créés, au moins un signal détectable indiquant le niveau d'expression d'au moins un produit du gène INK4a et d'au moins un autre signal détectable indiquant le niveau d'expression d'au moins un gène du marqueur de prolifération cellulaire.

36. Kit selon la revendication 35, **caractérisé en ce que** les produits du gène INK4a sont sélectionnés dans un groupe comprenant p16^{INK4a} et p14ARF.

37. Kit selon la revendication 35 ou 36, **caractérisé en ce que** les produits du gène du marqueur de prolifération cellulaire sont sélectionnés dans un groupe comprenant CDC6, MCM3, MCM3, MCM4, MCM5, MCM6, MCM7, CDC7 protéine kinase, Dbf4, CDC14 protéine phosphatase, CDC45 et MCM10, Ki67, Ki-S2, PCNA ou POLD.

38. Kit selon les revendications 35 à 37 comprenant en outre au moins l'un des produits suivants:
a. une échantillon p16^{INK4a} pour réaliser une réaction de contrôle positive
b.une échantillon p14ARF pour réaliser une réaction de contrôle positive
c.une échantillon Ki67 pour réaliser une réaction de contrôle positive
d. une échantillon Ki-S2 pour réaliser une réaction de contrôle positive
e. une échantillon MCM5 pour réaliser une réaction de contrôle positive
f. une échantillon MCM2 pour réaliser une réaction de contrôle positive
g. une échantillon PCNA pour réaliser une réaction de contrôle positive
h. des réactifs pour détecter la présence ou absence et/ou le niveau de p16INK4a
i. des réactifs pour détecter la présence ou absence et/ou le niveau de p14ARF
j. des réactifs pour détecter la présence ou absence et/ou le niveau de Ki67
k. des réactifs pour détecter la présence ou absence et/ou le niveau de Ki-S2
l. des réactifs pour détecter la présence ou absence et/ou le niveau de MCM5
m. des réactifs pour détecter la présence ou absence et/ou le niveau de MCM2
n. des réactifs pour détecter la présence ou absence et/ou le niveau de PCNA
o. une ou plus échantillons de produits du gène INK4a pour réaliser des réactions de contrôle positives
p. une ou plus échantillons des produits du gène du marqueur de prolifération cellulaire pour réaliser des réactions de contrôle positives
q. un ou plus réactifs pour la détection de la présence ou absence et/ou du niveau d'autres produits du gène INK4a
r. un ou plus réactifs pour la détection de la présence ou absence et/ou du niveau d'autres produits du gène du marqueur de prolifération cellulaire
